(19) Europäisches Patentamt
European Patent Office
Office européen des brevets

(11) Publication number: **0 291 981 B1**

# (12) EUROPEAN PATENT SPECIFICATION

(45) Date of publication of patent specification: **14.04.93**

(51) Int. Cl.[5]: **C07K 7/06**, C07K 7/08, A61K 37/02

(21) Application number: **88108047.7**

(22) Date of filing: **19.05.88**

## (54) Cyclic anticoagulant peptides.

(30) Priority: **21.05.87 US 53204**

(43) Date of publication of application:
**23.11.88 Bulletin 88/47**

(45) Publication of the grant of the patent:
**14.04.93 Bulletin 93/15**

(84) Designated Contracting States:
**AT BE CH DE ES FR GB GR IT LI LU NL SE**

(56) References cited:

**BIOCHIMCA ET BIOPHYSICA ACTA, 957 (1988), pages 53-59; Elsevier, US: J.L. KRSTENANSKY et al.: "Design, Synthesis and antithrombin activity for conformationally restricted analogs of peptide anticoagulants based on the C-terminal region of the leech peptide, hirudin"**

(73) Proprietor: **MERRELL DOW PHARMACEUTICALS INC.**
**2110 East Galbraith Road**
**Cincinnati Ohio 45215-6300(US)**

(72) Inventor: **Krstenansky, John L.**
**3749 Ault Park**
**Cincinnati Ohio 45208(US)**
Inventor: **Mao, Simon J.T.**
**9373 Kentonsrun Court**
**Loveland Ohio 45140(US)**

(74) Representative: **Vossius & Partner**
**Siebertstrasse 4 P.O. Box 86 07 67**
**W-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

This invention relates to novel cyclic peptides which are useful anticoagulant agents.

Anticoagulants are useful therapeutic agents in the pharmacological treatment of, for example, acute deep venous thrombosis, pulmonary embolism, acute arterial embolization of the extremities, myocardial infarction, and disseminated intravascular coagulation. Prophylactic administration of anticoagulants is believed to prevent a recurrance of embolism in patients with rheumatic or arteriosclerotic heart disease and to prevent certain thromboembolic complications of surgery. Administration of anticoagulants has also been indicated in the treatment of coronary artery and cerebrovascular disease. Artrial thrombosis, particularly in arteries supplying the heart muscle and brain, is a leading cause of death.

Hirudin is a 65 residue polypeptide isolated from the salivary glands of leeches. It is an anticoagulant agent, which is a thrombin specific inhibitor. Although quite potent, clinical use of hirudin isolated from leech extracts seems unlikely because of its limited quantity, expense and allergic reactions which commonly follow administration of any foreign protein of this size.

Applicants have discovered a specific region of hirudin that is responsible, at least in part, for its anticoagulant activity. This region has been chemically synthesized and certain of its cyclic analogs appear to bind to the recognition site of thrombin but not the enzymatic cleavage site which is spatially separate. Binding of the synthetic peptides competitively prevents binding of the fibrinogen to the recognition site of thrombin, a prerequisite to fibrin production and clot formation. The peptides of this invention possess significant anticoagulant activity and their unusual ability to bind only to the recognition site without binding to the cleavage site of thrombin may allow for a scientifically interesting and therapeutically significant adjunct to anticoagulant therapy.

This invention relates to derivatives of Hirudin having the structural formula 1:

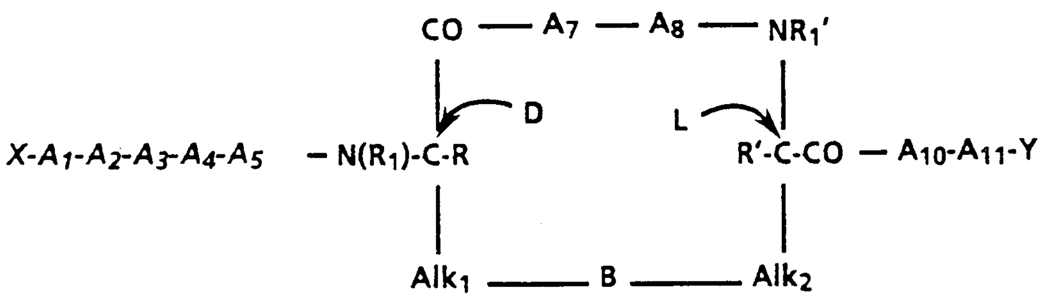

1

wherein

| | |
|---|---|
| X | is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl; |
| $A_1$ | is a bond or is a peptide containing from 1 to 5 residues of any amino acid; |
| $A_2$ | is Phe, SubPhe, β-(2- and 3-thienyl)alanine, β-(2- and 3-furanyl)alanine, β-(2-, 3-, and 4-pyridyl)alanine, β-(benzothien-2- and 3-yl)alanine, β-(1- and 2-naphthyl)-alanine, Tyr, or Trp; |
| $A_3$ | is Glu or Asp; |
| $A_4$ | is any amino acid; |
| $A_5$ | is Ile, Val, Leu, Nle, or Thr; |
| $A_7$ | is any amino acid; |
| $A_5$ | is any amino acid; |
| $A_{10}$ | is a lipophilic amino acid selected from Tyr, Tyr($SO_3H$), Trp, Phe, Leu, Nle, Ile, Val, His and Pro or is a dipeptide containing at least one of these lipophilic amino acids; |
| $A_{11}$ | is a bond or is a peptide fragment containing from one to five residues of any amino acid; |
| Y | is a carboxy terminal residue selected from OH, ($C_1$-$C_6$)alkoxy, amino, mono- or di-($C_1$-$C_4$)alkyl substituted amino, or benzylamino; |
| R, R′, $R_1$, and $R_1'$ | are each selected from a hydrogen or ($C_1$-$C_4$)alkyl group; |
| B | is selected from -S-, -S-S-, or -S-$Alk_3$-S-; and |
| $Alk_1$, $Alk_2$, and $Alk_3$ | are each selected from a ($C_1$-$C_8$)methylene or ethylene group; |

and wherein the "D" and "L" indicate that the stereochemistry of the indicated carbon is that corresponding to D-cysteine and L-cysteine, respectively, as well as the dimers of these peptide derivatives and their mixtures and the use of these peptide derivatives, dimers, and mixtures thereof as anticoagulant agents.

The following common abbreviations of the amino acids are used throughout this specification:

| | |
|---|---|
| Gly | - glycine |
| Ala | - alanine |
| Val | - valine |
| Leu | - leucine |
| Ile | - isoleucine |
| Pro | - proline |
| Phe | - phenylalanine |
| Trp | - tryptophan |
| Met | - methionine |
| Ser | - serine |
| Thr | - threonine |
| Cys | - cysteine |
| Tyr | - tyrosine |
| Asn | - asparagine |
| Gln | - glutamine |
| Asp | - aspartic acid |
| Glu | - glutaminc acid |
| Lys | - lysine |
| Arg | - arginine |
| His | - histidine |
| Nle | - norleucine |
| Hyp | - hydroxyproline |
| 3,4-dehydroPro | - 3,4-dehydroproline |
| Tyr($SO_3H$) | - tyrosine sulfate |
| Pgl | - phenylglycine |
| NMePgl | - N-methyl-phenylglycine |
| Sar | - sarcocine (N-methylglycine) |
| pSubPhe | - para substituted phenylalanine |
| SubPhe | - substituted phenylalanine |
| DAla | - D-alanine |
| Ac | - acetyl |
| Suc | - succinyl |
| pClPhe | - para-chloro-phenylalanine |
| p$NO_2$Phe | - para-nitro-phenylalanine |
| Pen | - penicillamine ($\beta$,$\beta$-dimethylcysteine) |
| DCys | - D-cysteine |

An alkyl group and the alkyl portion of an alkoxy group is taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl. An acyl group of from 2 to 10 carbon atoms is taken to include straight, branched, cyclic, saturated and unsaturated acyl groups having 1 or 2 carbonyl moieties per group, for example, acetyl, benzoyl and succinyl. The term "a ($C_1$-$C_8$)methylene or ethylene group" refers to a bivalent group derived from an acyclic or cyclic, saturated or unsaturated alkyl group of from 1 to 8 carbon atoms by conceptual removal of two hydrogen atoms from one of the carbon atoms or from two of the adjacent carbon atoms of the alkyl group. Examples of the ($C_1$-$C_8$)-methylene or ethylene groups of this invention are methylene or methylidene ($-CH_2-$), ethylidene ($CH_3CH<$), 1-methylethylidene ($CH_3C(CH_3)<$), 1-methylpropylidene or sec-butylidene ($CH_3CH_2C(CH_3)<$), 2,2-dimethylpropylidene or neopentylidene ($CH_3C(CH_3)_2CH<$), ethylene or dimethylene ($-CH_2CH_2-$), methylethylene ($-CH_2CH(CH_3)-$), ethylethylene ($-CH_2CH(C_2H_5)-$), ethenylene or vinylene ($-CH=CH-$), 1,1-ethenylidene ($CH_2=C<$), 1,1-cyclohexylidene ($C_6H_{10}<$), and 1,2-cyclopentylidene ($C_5H_8<$). A halogen group is a fluoro, chloro, bromo or iodo group.

The term "any amino acid" as used herein includes the naturally occurring amino acids as well as other "non-protein" $\alpha$-amino acids commonly utilized by those in the peptide chemistry arts when preparing synthetic analogs of naturally occurring peptides. The naturally occurring amino acids are glycine, alanine, valine, leucine, isoleucine, serine, methionine, threonine, phenylalanine, tyrosine, tryptophan, cysteine,

proline, histidine, aspartic acid, asparagine, glutamic acid, glutamine, arginine, ornithine, and lysine. Examples of "non-protein" α-amino acids are norleucine, norvaline, alloisoleucine, homoarginine, thiaproline, dehydroproline, hydroxyproline (Hyp), homoserine, cyclohexylglycine (Chg), α-amino-n-butyric acid (Aba), cyclohexylalanine (Cha), aminophenylbutyric acid (Pba), phenylalanines mono- or di-substituted at the ortho, meta, or para position of the phenyl moiety with ($C_1$-$C_4$) alkyl, ($C_1$-$C_4$) alkoxy, halogen, or nitro groups or with a methylenedioxy group, β-(2- and 3-thienyl)alanine, β-(2- and 3-furanyl)alanine, β-(2-, 3-, and 4-pyridyl)alanine, β-(benzothienyl-2- and 3-yl)alanine, β-(1- and 2-naphthyl)alanine, O-alkylated derivates of serine, threonine, or tyrosine, S-alkylated cysteine, the O-sulfate ester of tyrosine, 3,5-diiodotyrosine and the D-isomers of the naturally occurring amino acids.

The term "lipophilic amino acid" includes Tyr, Tyr($SO_3H$), Phe, Leu, Nle, Ile, Val, His, and Pro.

The natural amino acids with the exception of glycine, contain a chiral carbon atom. Unless otherwise specifically indicated, the optically active amino acids, referred to herein, are of the L-configuration. For example, any of the amino acids of the $A_1$ or $A_{10}$ group can be D- or L-configuration. As is customary, the structure of peptides written out herein is such that the amino terminal end is on the left side of the chain and the carboxy terminal end is on the right side of the chain.

The term "dimers" is intended to mean those peptides which result from the linking of two seperate linear peptides during the cyclization step either in a head to head or head to tail fashion. In the course of performing the desired internal cyclization via the "B" group, some of the linear peptide starting material will link with another linear peptide starting material rather then with itself. The resulting product is a "dimer" in the sense that it is made up of two of the linear starting peptides but is not a dimer in the sense that the molecular formula of the dimer is exactly two times the molecular formula of the monomer. A dimer of the peptide derivatives of this invention will have the structural formula:

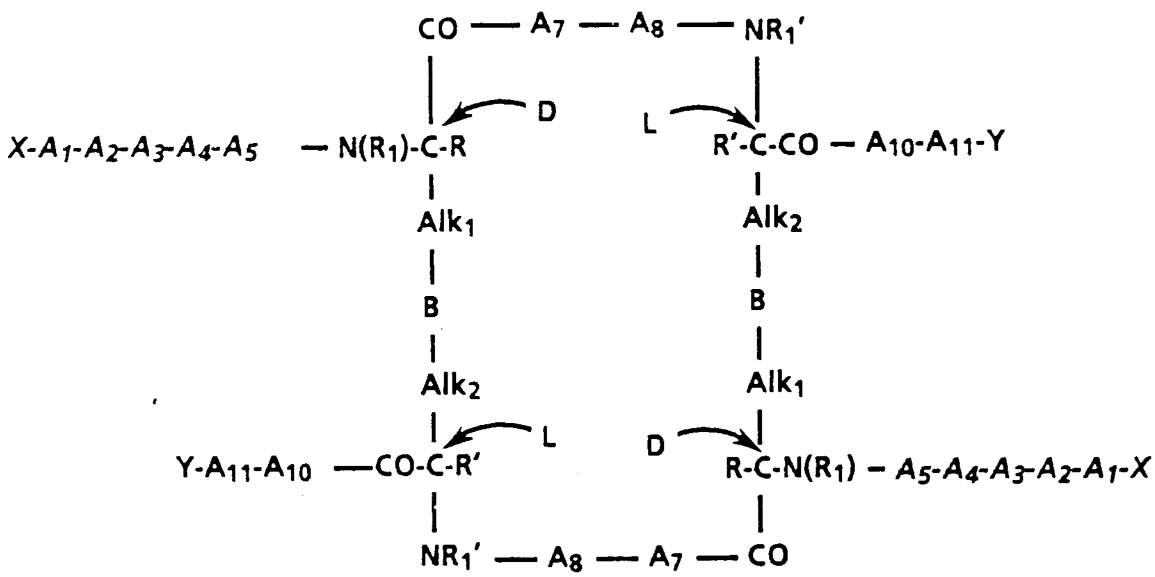

HEAD TO TAIL DIMER

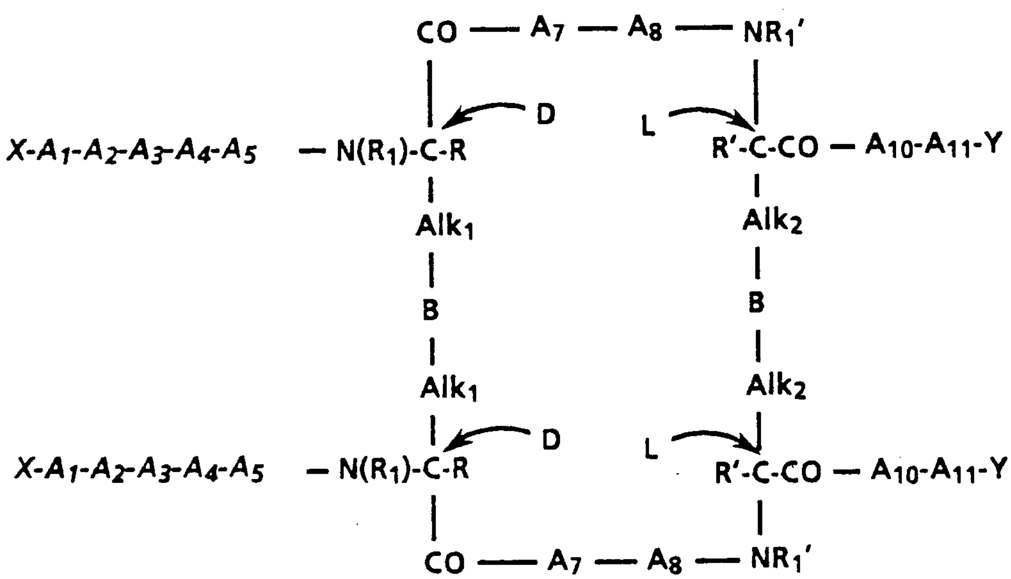

HEAD TO HEAD DIMER

wherein the substitutents are as defined above for structure 1. Throughout this disclosure, reference to the peptide derivatives includes the dimers and mixtures unless the context requires otherwise. While the mixtures of the monomer and dimer resulting from the cyclization step can be readily seperated by means well-known to those skilled in the art, the mixtures can be utilized in the antithrombotic compositions of this invention without separation.

The polypeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic,

phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N′-dibenzylethylenediamine, dihydroabietylamine, N-(lower)alkylpiperidine, and any other suitable amine.

As with any generic group of chemical compounds, certain groups are preferred. Applicants prefer those peptide derivatives of formula 1 wherein

X is hydrogen, acetyl, or succinyl.

Also preferred are those formula 1 compounds wherein

$A_1$ is

-His-Asn-Asp-Gly-Asp-,
-Asn-Asp-Gly-Asp-,
-Asp-Gly-Asp-,
-Gly-Asp-,
-Asp-,

or a bond.

| | |
|---|---|
| $A_2$ | is preferably Phe, $\beta$-(2- or 3-thienyl)alanine, Tyr, Trp, or pClPhe,; |
| $A_3$, | Glu; |
| $A_4$, | Glu, Asp, Pro, or Ala; |
| $A_5$, | Ile; |
| $A_7$, | Glu, Asp, or Ala; |
| $A_5$, | Glu or Asp; |
| $A_{10}$, | Leu; |
| $A_{11}$, | Pro, Gln, Asp, or Asp-Glu; |
| $Alk_1$ and $Alk_2$, | each a methylene group; |
| Y, | OH or $NH_2$; and |
| B, | -S-S-. |

Especially preferred are those peptide derivatives of formula 1 wherein either X is acetyl and $A_1$ is Gly-Asp or Asp or X is succinyl an $A_1$ is a bond and wherein

| | |
|---|---|
| $A_2$, | is Phe, $\beta$-(2-thienyl)alanine, or Tyr; |
| $A_3$, | Glu; |
| $A_4$, | Glu or Pro; |
| $A_5$, | Ile; |
| $A_7$, | Glu; |
| $A_5$, | Glu or Asp; |
| $A_9$, | Tyr, Ala-Tyr or Glu-Leu; |
| $A_{10}$, | -Leu-Gln- or -Asp-Glu; |
| $A_{11}$, | Pro, Gln, Asp, or Asp-Glu; |
| R, R′, $R_1$, and $R_1'$, | each hydrogen; |
| $Alk_1$ and $Alk_2$, | each a methylene group; |
| B, | -S-S-; and |
| Y, | OH. |

The peptides of this invention can be prepared by a variety of procedures readily known to those skilled in the art. Such procedures include the solid phase sequential and block synthesis, gene cloning and combinations of these techniques. The solid phase sequential procedure can be performed using established automated methods such as by use of an automated peptide sythesizer. In this procedure an $\alpha$-amino protected amino acid is bound to a resin support. The resin support employed can be any suitable resin conventionally employed in the art for the solid phase preparation of polypeptides, preferably polystyrene which has been cross-linked with from 0.5 to about 3 percent divinyl benzene, which has been either chloromethylated or hydroxymethylated to provide sites for ester formation with the initially introduced $\alpha$-amino protected amino acid.

An example of a hydroxymethyl resin is described by Bodanszky et al., Chem. Ind. (London) 38, 1597-98 (1966). A chloromethylated resin is commercially available from Bio Rad Laboratories, Richmond, California, and the preparation of such a resin is described by Stewart et al., "Solid Phase Peptide Synthesis" (Freeman & Co., San Francisco 1969), Chapter 1, pp. 1-6. The protected amino acid can be bound to the resin by the procedure of Gisin, Helv. Chem Acta, 56, 1476 (1973). Many resin bound, protected amino acids are commercially available. As an example, to prepare a polypeptide of this invention wherein the carboxy terminal end is a Thr residue, a tert-butyloxycarbonyl (Boc) protected Thr bound to benzylated, hydroxymethylated phenylacetamidomethyl (PAM) resin can be used and is commercially available.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0°C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitrophenylsulfenyl, tritylsulfenyl, o-nitrophenoxyacetyl and α-chlorobutyryl; (2) aromatic urethan type protecting groups such as benzyloxycarbonyl and substituted benzyloxycarbonyl, such as p-chlorobenzyloxycarbonyl, p-nitrobenzylcarbonyl, p-bromobenzyloxycarbonyl, p-methoxybenzyloxycarbonyl, 1-(p-biphenylyl)-1-methylethoxycarbonyl, α, α-dimethyl-3,5-dimethoxybenzyloxycarbonyl and benzhydryloxycarbonyl; (3) aliphatic urethan protecting groups such as tert-butyloxycarbonyl (Boc), diisopropylmethoxycarbonyl, isopropyloxycarbonyl, ethoxycarbonyl and allyloxycarbonyl; (4) cycloalkyl urethan type protecting groups such as cyclopentyloxycarbonyl, adamantyloxycarbonyl and cyclohexyloxycarbonyl; (5) thio urethan type protecting groups such as phenylthiocarbonyl; (6) alkyl type protecting groups such as triphenylmethyl (trityl) and benzyl; and (7) trialkylsilane groups such as trimethylsilane. The preferred α-amino protecting group is tert-butyloxycarbonyl.

The selection of an appropriate coupling reagent is within the skill of the art. A particularly suitable coupling reagent where the amino acid to be added is Gln, Asn or Arg is N,N′-diisopropylcarbodiimide and 1-hydroxybenzotriazole. The use of these reagents prevents nitrile and lactam formation. Other coupling agents are (1) carbodiimides (e.g., N,N′-dicyclohexylcarbodiimide and N-ethyl-N′-(γ-dimethylaminopropylcarbodiimide);(2) cyanamides (e.g., N,N-dibenzylcyanamide); (3) ketenimines; (4) isoxazolium salts (e.g., N-ethyl-5-phenyl-isoxazolium-3′-sulfonate); (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N′-carbonyldiimidazole and N,N-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-O-Ala-Boc) and (8) nitrogen containing heterocyclic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a solution of dimethyl sulfide, p-cresol and thiocresol in dilute aqueous hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain

protecting group is critical in that it must be one which is not removed by cleavage during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxycarbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The preferred protecting group is benzyl.

These groups can be removed by procedures well known in the art. Typically protecting group removal is done after the peptide chain synthesis is complete but the protecting groups can be removed at any other appropriate time.

In general, the cyclized peptides are prepared from an appropriate linear derivative either prior to or after removal of the linear peptide from the solid support. The compounds of formula 1 wherein B is a -S-S- group are prepared from the corresponding free sulfhydryl-containing, linear peptides by well known oxidative coupling technics such as by oxidizing the linear peptide with potassium ferricyanide described in, for example, Stewart et al., "Solid Phase Peptide Synthesis" (Freeman & Co., San Francisco 1969), Chapter 1, p. 95. The compounds of Structure 1 wherein B is a -S-$Alk_3$-S- group and $Alk_3$ is a ($C_1$-$C_8$)ethylene group can be prepared from the free sulfhydryl-containing linear peptides by reaction with a 1,2-dibromo derivative of an appropriate acyclic or cyclic, saturated or unsaturated alkyl in a manner analogous to that described in H. I. Mosberg and J. R. Omnaas, J. Amer. Chem. Soc. 107, 2986-2987 (1985). The compounds of formula 1 wherein B is a -S-$Alk_3$-S- group and $Alk_3$ is a ($C_1$-$C_8$)methylene group are prepared by reaction of the free sulfhydryl-containing linear peptide with an appropriate acyclic or cyclic, saturated or unsaturated alkyl ketone or aldehyde in a manner analogous to that described in J. Amer. Chem. Soc. 76, 1945 (1954). The preparation of those compounds of formula 1 wherein B is an -S- group can be accomplished in the manner set forth in K. Jost, Collect. Czech. Chem. Commun. 36, 218 (1971) and in United States Patent Number 4161521.

The anticoagulant dose of a peptide derivative of this invention is from 0.2 mg/kg to 250 mg/kg of patient body weight per day depending on the patient, the severity of the thromobotic condition to be treated and the peptide derivative selected. The suitable dose for a particular patient can be readily determined. Preferably from 1 to 4 daily doses would be administered typically with from 5 mg to 100 mg of active compound per dose.

Anticoagulant therapy is indicated for the treatment and prevention of a variety of thrombotic conditions, particularly coronary artery and cerebrovascular disease. Those experienced in this field are readily aware of the circumstances requiring anticoagulant therapy. The term "patient" used herein is taken to mean mammals such as primates, including humans, sheep, horses, cattle, pigs, dogs, cats, rats and mice.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containg a peptide derivative of this invention in a spray or dry powder form.

For parentral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

This invention is illustrated by the following, nonlimiting examples.

EXAMPLE 1

## Preparation of

H-Gly-Asp-Phe-Glu-Glu-Ile-DCys-Glu-Glu-Cys-Leu-Gln-OH

The peptide was snythesized by solid-phase methods using 0.1 mmol of a 0.66 mmol/g Boc-Gln-PAM resin. Double symmetrical anhydride couplings were performed with 2.0 mmol Nα-Boc-amino acid (Peptides International) except in the case of Boc-Gln, which was coupled by the DCC/HOBT method. The side chain protection utilized was: Asp(Chx), Cys(pMeBzl), Glu(Bzl). Upon completion of the synthesis the Nα-Boc protection was removed with 50% trifluoroacetic acid in methylene chloride. The resin was washed three times with methylene chloride, neutralized with three washings of 10% diisopropylethylamine in methylene chloride, washed three times with methylene chloride, acetylated with N-acetylimidazole in methylene chloride, washed three times with methylene chloride, and dried in vacuo. The peptide was deprotected and cleaved from the resin with water and the pH adjusted to 8.5 with ammonium hydroxide. Potassium ferricyanide (0.01 N) was added to the solution until a yellow color persisted. The solution was stirred for 30 minutes, then the pH was adjusted to between 4 and 5 with acetic acid. The mixture was then stirred with Bio-Rad® AG3-X4A ion exchange resin for 2 hours. The mixture was filtered and the filtrate lyophilized.

The peptide was purified by desalting on a 92 x 2.6 cm Sephadex® G-15 column in 5% aqueous acetic acid and lyophilized. Preparative HPLC was performed on a $C^{18}$ Vydac® 218TP1010 (250 x 10 mm) column with acetonitrile in 0.1% aqueous trifluoroactic acid at 5 ml/min. The major peak was collected and lyophilized leaving the desired product. Homogeneity was determined by HPLC and TLC. HPLC Vydac 218TP54 (250 x 4.6 mm) $C^{18}$ column, 2 ml/min, $t_o$ = 1.8 min: time of elution with a 25-50% acetonitrile in 0.1% trifluoroacetic acid linear gradient at 1%/min. (HPLC) is 5.5 min. FAB-MS: (M + H) 1411.7 ± 1 mμ (calcd. 1410). Amino acid analysis: (6N HCl hydrolysis; 24 hr. at 106 ° C), see Table 1, 57% peptide content by weight.

TABLE 1

| EXAMPLE NO. | Amino Acid Analysis (6N HCl Hydrolysis; 24 Hrs at 106° C) | | | | | | | | | | |
|---|---|---|---|---|---|---|---|---|---|---|---|
| | His | Asx | Ser | Glx | Pro | Ala | Gly | Ile* | Leu | Tyr | Phe |
| 1 | - | 1.00(1) | - | 5.05(5) | - | - | 0.95(1) | 0.60(1) | 1.00(1) | - | 1.02(1) |

* Conversion to allo-Ile not quantitated during hydrolysis.

## Claims

**Claims for the following Contracting States : AT, BE, CH, DE, FR, GB, IT, LI, LU, NL, SE**

**1.** A peptide derivative of the general formula 1:

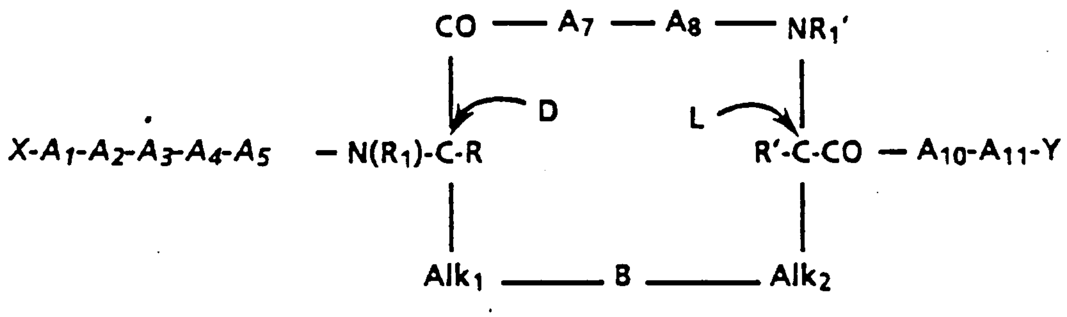

1

wherein

| | |
|---|---|
| X | is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl; |
| $A_1$ | is a bond or is a peptide containing from 1 to 5 residues of any amino acid; |
| $A_2$ | is Phe, Substituted phenylalanine (SubPhe), $\beta$-(2- and 3-thienyl)alanine, $\beta$-(2- and 3-furanyl)alanine, $\beta$-(2-, 3-, and 4-pyridyl)alanine, $\beta$-(benzothien-2- and 3-yl)alanine, $\beta$-(1- and 2-naphthyl)alanine, Tyr, or Trp; |
| $A_3$ | is Glu or Asp; |
| $A_4$ | is any amino acid; |
| $A_5$ | is Ile, Val, Leu, Nle, or Thr; |
| $A_7$ | is any amino acid; |
| $A_5$ | is Glu or Asp; |
| $A_{10}$ | is a lipophilic amino acid selected from Tyr, Tyr($SO_3H$), Trp, Phe, Leu, Nle, Ile, Val, His, and Pro or is a dipeptide containing at least one of these lipophilic amino acids; |
| $A_{11}$ | is a bond or is a peptide fragment containing from one to five residues of any amino acid; |
| Y | is a carboxy terminal residue selected from OH, ($C_1$-$C_6$)alkoxy, amino, mono- or di-($C_1$-$C_4$)alkyl substituted amino, or benzylamino; |
| R, R′, $R_1$, and $R_1$′ | are each selected from a hydrogen or ($C_1$-$C_4$)alkyl group; |
| B | is selected from -S-, -S-S-, or -S-$Alk_3$-S-; |
| $Alk_1$, $Alk_2$, and $Alk_3$ | are each selected from a ($C_1$-$C_8$)methylene or ethylene group; |

and wherein "D" and "L" indicate that the stereochemistry of the indicated carbon is that corresponding to D-cysteine and L-cysteine, respectively, and the pharmaceutically acceptable salts thereof.

**2.** A peptide derivative of claim 1 wherein $A_2$ is Phe, $\beta$-(2- or 3-thienyl)alanine, or Tyr.

**3.** A peptide derivative of claim 1 or 2 wherein $A_3$ is Glu.

**4.** A peptide derivative of anyone of claims 1 to 3 wherein $A_4$ is Glu.

**5.** A peptide derivative of anyone of claims 1 to 4 wherein $A_5$ is Ile.

**6.** A peptide derivative of anyone of claims 1 to 5 wherein $A_7$ is Glu or Ala.

**7.** A peptide derivative of anyone of claims 1 to 6 wherein $A_5$ is Glu or Asp.

**8.** A peptide derivative of anyone of claims 1 to 7 wherein $A_{10}$ is Leu.

**9.** A peptide derivative of anyone of claims 1 to 8 wherein $A_{11}$ is Pro, Gln, Asp, or Asp-Gln.

**10.** A peptide derivative of anyone of claims 1 to 9 wherein X is H, acetyl, or succinyl.

**11.** A peptide derivative of anyone of claims 1 to 10 wherein Y is OH or $NH_2$.

**12.** A peptide derivative of anyone of claims 1 to 11 wherein R, $R'$, $R_1$ , $R_1'$ are each a hydrogen.

**13.** A peptide derivative of anyone of claims 1 to 12 wherein B is the group -S-S-.

**14.** A peptide derivative of anyone of claims 1 to 13 wherein $Alk_1$ and $Alk_2$ are each a methylene group of the formula $-(CH_2)-$.

**15.** A dimer of a peptide derivative of anyone of claims 1 to 14 or a mixture of the dimer and said peptide.

**16.** A process for the preparation of a peptide derivative of anyone of claims 1 to 14, of a dimer thereof, or of a mixture of the dimer and said polypeptide comprising preparing the free sulfhydryl-containing linear peptide by solid phase sequential or block synthesis, gene cloning, or a combination thereof and subsequently subjecting the linear peptide to an oxidative coupling.

**17.** A solid phase sequential or block synthesis process for the preparation of a peptide derivative of anyone of claims 1 to 14, of a dimer thereof or of a mixture of the dimer and said polypeptide comprising binding a suitably protected amino acid of formula $A_1$ having the meaning given in claim 1 to an activated resin support, subsequently binding the other alpha amino protected amino acids of formula $A_2$ to $A_{11}$ having the meaning given in the preceding claims to the terminal amino group of the growing peptidic chain which has meanwhile been exposed by removing its amino protecting group, and finally subjecting the linear peptide to an oxidative coupling.

**18.** A peptide derivative of anyone of claims 1 to 14, a dimer thereof, or a mixture of these compounds for use as a pharmaceutically active compound.

**19.** A peptide derivative of anyone of claims 1 to 14, a dimer thereof, or a mixture of these compounds for use as an anticoagulant.

**20.** A pharmaceutical composition containing an anticoagulant effective amount of a peptide derivative of anyone of claims 1 to 14, of a dimer thereof, or of a mixture of these compounds and optionally a pharmaceutically acceptable carrier and/or diluent.

**Claims for the following Contracting State: ES**

**1.** A process for preparing a peptide derivative of the formula

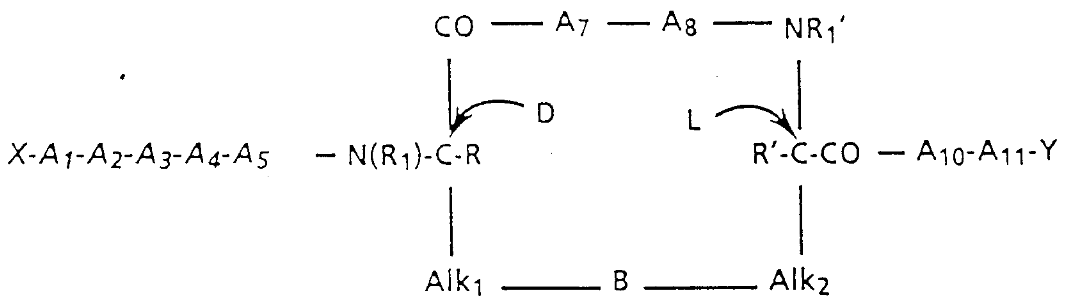

1

wherein

| | |
|---|---|
| X | is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl; |
| $A_1$ | is a bond or is a peptide containing from 1 to 5 residues of any amino acid; |

| | |
|---|---|
| $A_2$ | is Phe, Substituted phenylalanine (SubPhe), $\beta$-(2- and 3-thienyl)alanine, $\beta$-(2- and 3-furanyl)alanine, $\beta$-(2-, 3-, and 4-pyridyl)alanine, $\beta$-(benzothien-2- and 3-yl)alanine, $\beta$-(1- and 2-naphthyl)alanine, Tyr, or Trp; |
| $A_3$ | is Glu or Asp; |
| $A_4$ | is any amino acid; |
| $A_5$ | is Ile, Val, Leu, Nle, or Thr; |
| $A_7$ | is any amino acid; |
| $A_5$ | is Glu or Asp; |
| $A_{10}$ | is a lipophilic amino acid selected from Tyr, Tyr($SO_3H$), Trp, Phe, Leu, Nle, Ile, Val, His, and Pro or is a dipeptide containing at least one of these lipophilic amino acids; |
| $A_{11}$ | is a bond or is a peptide fragment containing from one to five residues of any amino acid; |
| Y | is a carboxy terminal residue selected from OH, ($C_1$-$C_6$)alkoxy, amino, mono- or di-($C_1$-$C_4$)alkyl substituted amino, or benzylamino; |
| R, R′, $R_1$, and $R_1'$ | are each selected from a hydrogen or ($C_1$-$C_4$)alkyl group; |
| B | is selected from -S-, -S-S-, or -S-$Alk_3$-S-; |
| $Alk_1$, $Alk_2$, and $Alk_3$ | are each selected from a ($C_1$-$C_8$)methylene or ethylene group; |

and wherein "D" and "L" indicate that the stereochemistry of the indicated carbon is that corresponding to D-cysteine and L-cysteine, respectively, comprising preparing the free sulfhydryl-containing linear peptide by solid phase sequential or block synthesis, gene cloning, or a combination thereof and subsequently subjecting the linear peptide to an oxidative coupling.

**2.** A solid phase sequential or block synthesis process for preparing a peptide derivative of the formula

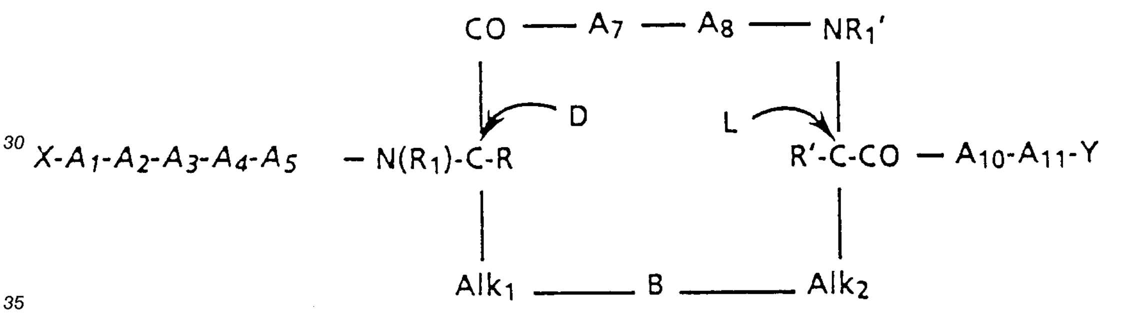

1

wherein

| | |
|---|---|
| X | is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl; |
| $A_1$ | is a bond or is a peptide containing from 1 to 5 residues of any amino acid; |
| $A_2$ | is Phe, SubPhe, $\beta$-(2- and 3-thienyl)alanine, $\beta$-(2- and 3-furanyl)alanine, $\beta$-(2-, 3-, and 4-pyridyl)alanine, $\beta$-(benzothien-2- and 3-yl)alanine, $\beta$-(1- and 2-naphthyl)alanine, Tyr, or Trp; |
| $A_3$ | is Glu or Asp; |
| $A_4$ | is any amino acid; |
| $A_5$ | is Ile, Val, Leu, Nle, or Thr; |
| $A_7$ | is any amino acid; |
| $A_5$ | is Glu or Asp; |
| $A_{10}$ | is a lipophilic amino acid selected from Tyr, Tyr($SO_3H$), Trp, Phe, Leu, Nle, Ile, Val, His, and Pro or is a dipeptide containing at least one of these lipophilic amino acids; |
| $A_{11}$ | is a bond or is a peptide fragment containing from one to five residues of any amino acid; |
| Y | is a carboxy terminal residue selected from OH, ($C_1$-$C_6$) alkoxy, amino, mono- or di-($C_1$-$C_4$)alkyl substituted amino, or benzylamino; |
| R, R′, $R_1$, and $R_1'$ | are each selected from a hydrogen or ($C_1$-$C_4$)alkyl group; |
| B | is selected from -S-, -S-S-, or -S-$Alk_3$-S-; |

Alk$_1$, Alk$_2$, and Alk$_3$ are each selected from a ($C_1$-$C_8$)methylene or ethylene group;

and wherein "D" and "L" indicate that the stereochemistry of the indicated carbon is that corresponding to D-cysteine and L-cysteine, respectively, comprising binding a suitably protected amino acid of formula $A_1$ to an activated resin support, subsequently binding the other alpha amino protected amino acids from $A_2$ to $A_{11}$ to the terminal amino group of the growing peptidic chain which has meanwhile been exposed by removing its amino protecting group, and finally subjecting the linear peptide to an oxidative coupling.

**3.** The process according to claim 1 or 2 which additionally comprises the formation of a dimer of the obtained peptide derivative.

**4.** A process of anyone of claims 1 to 3 wherein $A_2$ is Phe, $\beta$-(2- or 3-thienyl)alanine, or Tyr.

**5.** A process of anyone of claims 1 to 4 wherein $A_3$ is Glu.

**6.** A process of anyone of claims 1 to 5 wherein $A_4$ is Glu.

**7.** A process of anyone of claims 1 to 6 wherein $A_5$ is Ile.

**8.** A process of anyone of claims 1 to 7 wherein $A_7$ is Glu or Ala.

**9.** A process of anyone of claims 1 to 8 wherein $A_5$ is Glu or Asp.

**10.** A process of anyone of claims 1 to 9 wherein $A_{10}$ is Leu.

**11.** A process of anyone of claims 1 to 10 wherein $A_{11}$ is Pro, Gln, Asp, or Asp-Gln.

**12.** A process of anyone of claims 1 to 11 wherein X is H, acetyl, or succinyl.

**13.** A process of anyone of claims 1 to 12 wherein Y is OH or $NH_2$.

**14.** A process of anyone of claims 1 to 13 wherein R, R$'$, $R_1$, $R_1'$ are each hydrogen.

**15.** A process of anyone of claims 1 to 14 wherein B is the group -S-S-.

**16.** A process of anyone of claims 1 to 15 wherein Alk$_1$ and Alk$_2$ are each a methylene group of the formula $-(CH_2)-$.

**17.** Use of a compound obtainable according to the process of anyone of claims 1 to 16 for the preparation of a pharmaceutical composition.

**18.** Use of a compound obtainable according to the process of anyone of claims 1 to 16 for the preparation of an anticoagulant.

**Claims for the following Contracting State : GR**

**1.** A peptide derivative of the general formula 1:

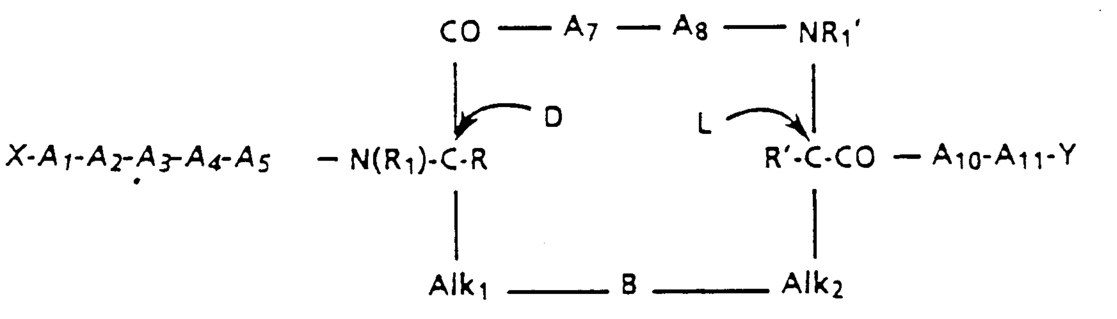

1

wherein

| | |
|---|---|
| X | is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl; |
| $A_1$ | is a bond or is a peptide containing from 1 to 5 residues of any amino acid; |
| $A_2$ | is Phe, Substituted phenylalanine (SubPhe), $\beta$-(2- and 3-thienyl)alanine, $\beta$-(2- and 3-furanyl)alanine, $\beta$-(2-, 3-, and 4-pyridyl)alanine, $\beta$-(benzothien-2- and 3-yl)alanine, $\beta$-(1- and 2-naphthyl)alanine, Tyr, or Trp; |
| $A_3$ | is Glu or Asp; |
| $A_4$ | is any amino acid; |
| $A_5$ | is Ile, Val, Leu, Nle, or Thr; |
| $A_7$ | is any amino acid; |
| $A_5$ | is Glu or Asp; |
| $A_{10}$ | is a lipophilic amino acid selected from Tyr, Tyr($SO_3H$), Trp, Phe, Leu, Nle, Ile, Val, His, and Pro or is a dipeptide containing at least one of these lipophilic amino acids; |
| $A_{11}$ | is a bond or is a peptide fragment containing from one to five residues of any amino acid; |
| Y | is a carboxy terminal residue selected from OH, ($C_1$-$C_6$)alkoxy, amino, mono- or di-($C_1$-$C_4$)alkyl substituted amino, or benzylamino; |
| R, R′, $R_1$, and $R_1'$ | are each selected from a hydrogen or ($C_1$-$C_4$)alkyl group; |
| B | is selected from -S-, -S-S-, or -S-$Alk_3$-S-; |
| $Alk_1$, $Alk_2$, and $Alk_3$ | are each selected from a ($C_1$-$C_8$)methylene or ethylene group; |

and wherein "D" and "L" indicate that the stereochemistry of the indicated carbon is that corresponding to D-cysteine and L-cysteine, respectively/and the pharmaceutically acceptable salts thereof.

**2.** A peptide derivative of claim 1 wherein $A_2$ is Phe, $\beta$-(2- or 3-thienyl)alanine, or Tyr.

**3.** A peptide derivative of claim 1 or 2 wherein $A_3$ is Glu.

**4.** A peptide derivative of anyone of claims 1 to 3 wherein $A_4$ is Glu.

**5.** A peptide derivative of anyone of claims 1 to 4 wherein $A_5$ is Ile.

**6.** A peptide derivative of anyone of claims 1 to 5 wherein $A_7$ is Glu or Ala.

**7.** A peptide derivative of anyone of claims 1 to 6 wherein $A_5$ is Glu or Asp.

**8.** A peptide derivative of anyone of claims 1 to 7 wherein $A_{10}$ is Leu.

**9.** A peptide derivative of anyone of claims 1 to 8 wherein $A_{11}$ is Pro, Gln, Asp, or Asp-Gln.

**10.** A peptide derivative of anyone of claims 1 to 9 wherein X is H, acetyl, or succinyl.

**11.** A peptide derivative of anyone of claims 1 to 10 wherein Y is OH or $NH_2$.

**12.** A peptide derivative of anyone of claims 1 to 11 wherein R, R′, $R_1$, $R_1'$ are each a hydrogen.

**13.** A peptide derivative of anyone of claims 1 to 12 wherein B is the group -S-S-.

**14.** A peptide derivative of anyone of claims 1 to 13 wherein $Alk_1$ and $Alk_2$ are each a methylene group of the formula -($CH_2$)-.

**15.** A dimer of a peptide derivative of anyone of claims 1 to 14 or a mixture of the dimer and said peptide.

**16.** A process for the preparation of a peptide derivative of anyone of claims 1 to 14, of a dimer thereof, or of a mixture of the dimer and said polypeptide comprising preparing the free sulfhydryl-containing linear peptide by solid phase sequential or block synthesis, gene cloning, or a combination thereof and

subsequently subjecting the linear peptide to an oxidative coupling.

**17.** A solid phase sequential or block synthesis process for the preparation of a peptide derivative of anyone of claims 1 to 14, of a dimer thereof, or of a mixture of the dimer and said polypeptide comprising binding a suitably protected amino acid of formula $A_1$ having the meaning given in claim 1 to an activated resin support, subsequently binding the other alpha amino protected amino acids of formula $A_2$ to $A_{11}$ having the meaning given in the preceding claims to the terminal amino group of the growing peptidic chain which has meanwhile been exposed by removing its amino protecting group, and finally subjecting the linear peptide to an oxidative coupling.

**18.** A peptide derivative of anyone of claims 1 to 14, a dimer thereof, or a mixture of these compounds for use as a pharmaceutically active compound.

**19.** A peptide derivative of anyone of claims 1 to 14, a dimer thereof, or a mixture of these compounds for use as an anticoagulant.

**Patentansprüche**
**Patentansprüche für folgende Vertragsstaaten : AT, BE, CH, DE, FR, GB, IT, LI, LU, NL, SE**

**1.** Peptidderivat der allgemeinen Formel 1:

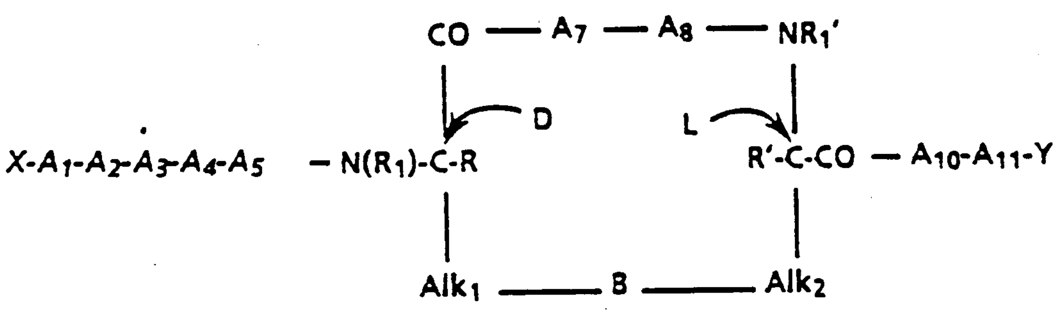

1

in der

| | |
|---|---|
| X | einen Rest an der endständigen Aminogruppe darstellt, ausgewählt aus einem Wasserstoffatom, einem oder zwei Alkylresten mit 1 bis 6 Kohlenstoffatomen, einem oder zwei Acylresten mit 2 bis 10 Kohlenstoffatomen, der Carbobenzyloxy- oder tert-Butyloxycarbonylgruppe; |
| $A_1$ | eine Bindung oder ein Peptid mit 1 bis 5 Aminosäureresten ist; |
| $A_2$ | Phe, substituiertes Phenylalanin (SubPhe), $\beta$-(2-und 3-Thienyl)alanin, $\beta$-(2- und 3-Furanyl)alanin, $\beta$-(2-, 3- und 4-Pyridyl)alanin, $\beta$-(Benzothien-2- und 3-yl)alanin, $\beta$-(1- und 2-Naphthyl)alanin, Tyr oder Trp ist; |
| $A_3$ | Glu oder Asp ist; |
| $A_4$ | irgendeine Aminosäure ist; |
| $A_5$ | Ile, Val, Leu, Nle oder Thr ist; |
| $A_7$ | irgendeine Aminosäure ist; |
| $A_5$ | Glu oder Asp ist; |
| $A_{10}$ | eine lipophile Aminosäure, ausgewählt aus Tyr, Tyr($SO_3H$), Trp, Phe, Leu, Nle, Ile, Val, His und Pro, oder ein Dipeptid, das mindestens eine dieser lipophilen Aminosäuren enthält, ist; |
| $A_{11}$ | eine Bindung oder ein Peptidfragment mit 1 bis 5 Resten irgendwelcher Aminosäuren ist; |
| Y | einen Rest an der endständigen Carboxygruppe darstellt, ausgewählt aus -OH, einem ($C_1$-$C_6$)Alkoxy-, Amino-, Mono- oder Di-($C_1$-$C_4$)alkylsubstituierten Amino- oder Benzylaminorest; |
| R, R', $R_1$ und $R_1$' | jeweils ausgewählt sind aus einem Wasserstoffatom oder einem ($C_1$-$C_4$)-Alkylrest; |
| B | ausgewählt ist aus den Gruppen -S-, -S-S- oder -S-$Alk_3$-S-; |
| $Alk_1$, $Alk_2$ und $Alk_3$ | jeweils ausgewählt sind aus einer ($C_1$-$C_8$)Methylen- oder Ethylengruppe; |

und in der "D" und "L" anzeigen, daß die Stereochemie des betreffenden Kohlenstoffatoms diejenige ist, die der von D-Cystein bzw. L-Cystein entspricht; und die pharmazeutisch verträglichen Salze davon.

**2.** Peptidderivat gemäß Anspruch 1, in dem $A_2$ Phe, $\beta$-(2-oder 3-Thienyl)alanin oder Tyr ist.

**3.** Peptidderivat gemäß den Ansprüchen 1 oder 2, in dem $A_3$ Glu ist.

**4.** Peptidderivat gemäß einem der Ansprüche 1 bis 3, in dem $A_4$ Glu ist.

**5.** Peptidderivat gemäß einem der Ansprüche 1 bis 4, in dem $A_5$ Ile ist.

**6.** Peptidderivat gemäß einem der Ansprüche 1 bis 5, in dem $A_7$ Glu oder Ala ist.

**7.** Peptidderivat gemäß einem der Ansprüche 1 bis 6, in dem $A_5$ Glu oder Asp ist.

**8.** Peptidderivat gemäß einem der Ansprüche 1 bis 7, in dem $A_{10}$ Leu ist.

**9.** Peptidderivat gemäß einem der Ansprüche 1 bis 8, in dem $A_{11}$ Pro, Gln, Asp oder Asp-Gln ist.

**10.** Peptidderivat gemäß einem der Ansprüche 1 bis 9, in dem X ein Wasserstoffatom, eine Acetyl- oder Bernsteinsäuregruppe ist.

**11.** Peptidderivat gemäß einem der Ansprüche 1 bis 10, in dem Y eine der Gruppen OH oder $NH_2$ ist.

**12.** Peptidderivat gemäß einem der Ansprüche 1 bis 11, in dem die Reste R, R', $R_1$, $R_1'$ jeweils ein Wasserstoffatom sind.

**13.** Peptidderivat gemäß einem der Ansprüche 1 bis 12, in dem B eine Gruppe -S-S- ist.

**14.** Peptidderivat gemäß einem der Ansprüche 1 bis 13, in dem $Alk_1$ und $Alk_2$ jeweils eine Methylengruppe der Formel $-(CH_2)-$ sind.

**15.** Dimer eines Peptidderivats gemäß einem der Ansprüche 1 bis 14 oder ein Gemisch aus dem Dimeren und besagtem Peptid.

**16.** Verfahren zur Herstellung eines Peptidderivats gemäß einem der Ansprüche 1 bis 14 oder eines Dimeren davon oder eines Gemisches des Dimeren und des besagten Polypeptids, das die Erzeugung des freien Sulfhydryl enthaltenden linearen Peptids durch eine Festphasen-Sequenz-oder Blocksynthese, Genclonierung, oder eine Kombination davon und anschließend die oxidative Kupplung des linearen Peptids umfaßt.

**17.** Festphasen-Sequenz- oder Blocksynthese-Verfahren zur Herstellung eines Peptidderivats gemäß einem der Ansprüche 1 bis 14 oder eines Dimeren davon oder eines Gemisches des Dimeren und des besagten Polypeptids, das das Anbinden einer in geeigneter Weise geschützten Aminosäure der Formel $A_1$, die die in Anspruch 1 angegebene Bedeutung hat, an einen aktivierten Harzträger, nachfolgend das Anbinden der anderen $\alpha$-Amino-geschützten Aminosäuren der Formeln $A_2$ bis $A_{11}$, die die in den vorangegangenen Ansprüchen angegebene Bedeutung haben, an die endständige Aminogruppe der wachsenden Peptidkette, die unterdessen durch Entfernen ihrer Aminoschutzgruppe freigesetzt wurde, und schließlich die oxidative Kupplung des linearen Peptids umfaßt.

**18.** Peptidderivat gemäß einem der Ansprüche 1 bis 14, ein Dimer davon oder ein Gemisch dieser Verbindungen zur Verwendung als Arzneistoff.

**19.** Peptidderivat gemäß einem der Ansprüche 1 bis 14, ein Dimer davon oder ein Gemisch dieser Verbindungen zur Verwendung als Antikoagulierungsmittel.

**20.** Arzneimittel, enthaltend eine koagulationshemmend wirksame Menge eines Peptidderivats gemäß einem der Ansprüche 1 bis 14, eines Dimeren davon oder eines Gemisches dieser Verbindungen und

gegebenenfalls einen pharmazeutisch verträglichen Träger und/oder ein Verdünnungsmittel.

**Patentansprüche für folgenden Vertragsstaat : ES**

**1.** Verfahren zur Herstellung eines Peptidderivats der allgemeinen Formel 1:

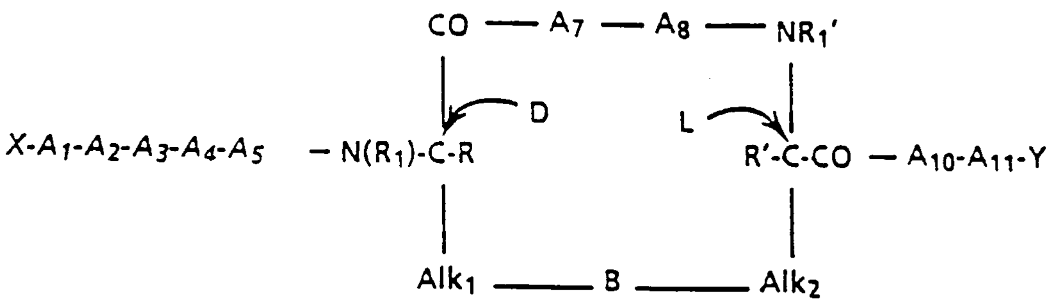

1

in der

| | |
|---|---|
| X | einen Rest an der endständigen Aminogruppe darstellt, ausgewählt aus einem Wasserstoffatom, einem oder zwei Alkylresten mit 1 bis 6 Kohlenstoffatomen, einem oder zwei Acylresten mit 2 bis 10 Kohlenstoffatomen, der Carbobenzyloxy- oder tert-Butyloxycarbonylgruppe; |
| $A_1$ | eine Bindung oder ein Peptid mit 1 bis 5 Aminosäureresten ist; |
| $A_2$ | Phe, substituiertes Phenylalanin (SubPhe), $\beta$-(2-und 3-Thienyl)alanin, $\beta$-(2- und 3-Furanyl)alanin, $\beta$-(2-, 3- und 4-Pyridyl)alanin, $\beta$-(Benzothien-2- und 3-yl)alanin, $\beta$-(1- und 2-Naphthyl)alanin, Tyr oder Trp ist; |
| $A_3$ | Glu oder Asp ist; |
| $A_4$ | irgendeine Aminosäure ist; |
| $A_5$ | Ile, Val, Leu, Nle oder Thr ist; |
| $A_7$ | irgendeine Aminosäure ist; |
| $A_5$ | Glu oder Asp ist; |
| $A_{10}$ | eine lipophiie Aminosäure, ausgewählt aus Tyr, Tyr($SO_3H$), Trp, Phe, Leu, Nle, Ile, Val, His und Pro, oder ein Dipeptid, das mindestens eine dieser iipophilen Aminosäuren enthält, ist; |
| $A_{11}$ | eine Bindung oder ein Peptidfragment mit 1 bis 5 Resten irgendwelcher Aminosäuren ist; |
| Y | einen Rest an der endständigen Carboxygruppe darstellt, ausgewählt aus -OH, einem ($C_1$-$C_6$)Alkoxy-, Amino-, Mono- oder Di-($C_1$-$C_4$)alkylsubstituierten Amino- oder Benzylaminorest; |
| R, R', $R_1$ und $R_1$' | jeweils ausgewählt sind aus einem Wasserstoffatom oder einem ($C_1$-$C_4$)-Alkylrest; |
| B | ausgewählt ist aus den Gruppen -S-, -S-S- oder -S-$Alk_3$-S-; |
| $Alk_1$, $Alk_2$ und $Alk_3$ | jeweils ausgewählt sind aus einer ($C_1$-$C_8$)Methylen- oder Ethylengruppe; |

und in der "D" und "L" anzeigen, daß die Stereochemie des betreffenden Kohlenstoffatoms diejenige ist, die der von D-Cystein bzw. L-Cystein entspricht; das die Erzeugung des freien Sulfhydryl enthaltenden linearen Peptids durch eine Festphasen-Sequenz- oder Blocksynthese, Genclonierung, oder eine Kombination davon und anschließend die oxidative Kupplung des linearen Peptids umfaßt.

**2.** Festphasen-Sequenz- oder Blocksynthese-Verfahren zur Herstellung eines Peptidderivats der Formel 1:

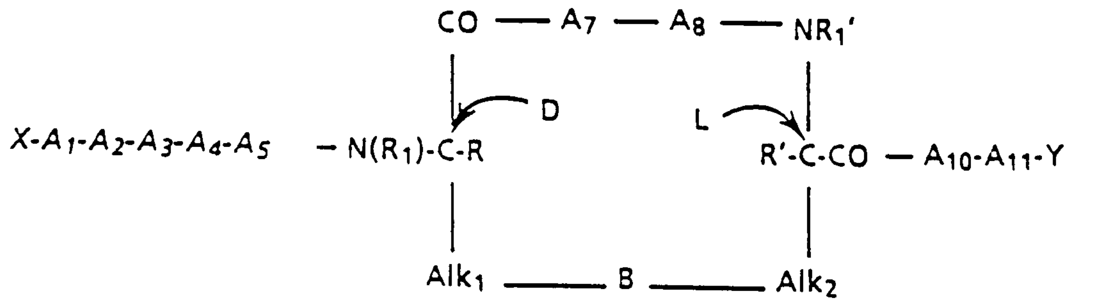

1

in der

| | |
|---|---|
| X | einen Rest an der endständigen Aminogruppe darstellt, ausgewählt aus einem Wasserstoffatom, einem oder zwei Alkylresten mit 1 bis 6 Kohlenstoffatomen, einem oder zwei Acylresten mit 2 bis 10 Kohlenstoffatomen, der Carbobenzyloxy- oder tert-Butyloxycarbonylgruppe; |
| $A_1$ | eine Bindung oder ein Peptid mit 1 bis 5 Aminosäureresten ist; |
| $A_2$ | Phe, substituiertes Phenylalanin (SubPhe), $\beta$-(2-und 3-Thienyl)alanin, $\beta$-(2- und 3-Furanyl)alanin, $\beta$-(2-, 3- und 4-Pyridyl)alanin, $\beta$-(Benzothien-2- und 3-yl)alanin, $\beta$-(1- und 2-Naphthyl)alanin, Tyr oder Trp ist; |
| $A_3$ | Glu oder Asp ist; |
| $A_4$ | irgendeine Aminosäure ist; |
| $A_5$ | Ile, Val, Leu, Nle oder Thr ist; |
| $A_7$ | irgendeine Aminosäure ist; |
| $A_5$ | Glu oder Asp ist; |
| $A_{10}$ | eine lipophile Aminosäure, ausgewählt aus Tyr, Tyr($SO_3H$), Trp, Phe, Leu, Nle, Ile, Val, His und Pro, oder ein Dipeptid, das mindestens eine dieser lipophilen Aminosäuren enthält, ist; |
| $A_{11}$ | eine Bindung oder ein Peptidfragment mit 1 bis 5 Resten irgendwelcher Aminosäuren ist; |
| Y | einen Rest an der endständigen Carboxygruppe darstellt, ausgewählt aus -OH, einem ($C_1$-$C_6$)Alkoxy-, Amino-, Mono- oder Di-($C_1$-$C_4$)alkylsubstituierten Amino- oder Benzylaminorest; |
| R, R', $R_1$ und $R_1$' | jeweils ausgewählt sind aus einem Wasserstoffatom oder einem ($C_1$-$C_4$)-Alkylrest; |
| B | ausgewählt ist aus den Gruppen -S-, -S-S- oder -S-$Alk_3$-S-; |
| $Alk_1$, $Alk_2$ und $Alk_3$ | jeweils ausgewählt sind aus einer ($C_1$-$C_8$)Methylen- oder Ethylengruppe; |

und in der "D" und "L" anzeigen, daß die Stereochemie des betreffenden Kohlenstoffatoms diejenige ist, die der von D-Cystein bzw. L-Cystein entspricht; das das Anbinden einer in geeigneter Weise geschützten Aminosäure der Formel $A_1$, die die in Anspruch 1 angegebene Bedeutung hat, an einen aktivierten Harzträger, nachfolgend das Anbinden der anderen $\alpha$-Amino-geschützten Aminosäuren der Formeln $A_2$ bis $A_{11}$, die die in den vorangegangenen Ansprüchen angegebene Bedeutung haben, an die endständige Aminogruppe der wachsenden Peptidkette, die unterdessen durch Entfernen ihrer Aminoschutzgruppe freigesetzt wurde, und schließlich die oxidative Kupplung des linearen Peptids umfaßt.

**3.** Verfahren gemäß den Ansprüchen 1 oder 2, das zusätzlich die Erzeugung eines Dimeren des erhaltenen Peptidderivats umfaßt.

**4.** Verfahren gemäß einem der Ansprüche 1 bis 3, in dem $A_2$ Phe, $\beta$-(2- oder 3-Thienyl)alanin oder Tyr ist.

**5.** Verfahren gemäß einem der Ansprüche 1 oder 4, in dem $A_3$ Glu ist.

**6.** Verfahren gemäß einem der Ansprüche 1 bis 5, in dem $A_4$ Glu ist.

**7.** Verfahren gemäß einem der Ansprüche 1 bis 6, in dem $A_5$ Ile ist.

**8.** Verfahren gemäß einem der Ansprüche 1 bis 7, in dem $A_7$ Glu oder Ala ist.

**9.** Verfahren gemäß einem der Ansprüche 1 bis 8, in dem $A_5$ Glu oder Asp ist.

**10.** Verfahren gemäß einem der Ansprüche 1 bis 9, in dem $A_{10}$ Leu ist.

**11.** Verfahren gemäß einem der Ansprüche 1 bis 10, in dem $A_{11}$ Pro, Gln, Asp oder Asp-Gln ist.

**12.** Verfahren gemäß einem der Ansprüche 1 bis 11, in dem X ein Wasserstoffatom, eine Acetyl- oder Bernsteinsäuregruppe ist.

**13.** Verfahren gemäß einem der Ansprüche 1 bis 12, in dem Y eine der Gruppen OH oder $NH_2$ ist.

**14.** Verfahren gemäß einem der Ansprüche 1 bis 13, in dem die Reste R, R', $R_1$, $R_1'$ jeweils ein Wasserstoffatom sind.

**15.** Verfahren gemäß einem der Ansprüche 1 bis 14, in dem B eine Gruppe -S-S- ist.

**16.** Verfahren gemäß einem der Ansprüche 1 bis 15, in dem $Alk_1$ und $Alk_2$ jeweils eine Methylengruppe der Formel $-(CH_2)-$ sind.

**17.** Verwendung einer Verbindung, die nach dem Verfahren gemäß einem der Ansprüche 1 bis 16 gewonnen wurde, zur Herstellung eines Arzneimittels.

**18.** Verwendung einer Verbindung, die nach dem Verfahren gemäß einem der Ansprüche 1 bis 16 gewonnen wurde, zur Herstellung eines Koagulationshemmers.

**Patentansprüche für folgenden Vertragsstaat : GR**

**1.** Peptidderivat der allgemeinen Formel 1:

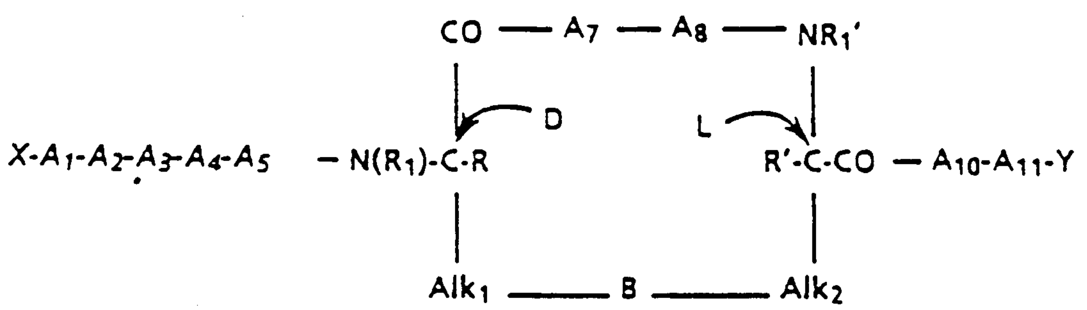

1

in der

| | |
|---|---|
| X | einen Rest an der endständigen Aminogruppe darstellt, ausgewählt aus einem Wasserstoffatom, einem oder zwei Alkylresten mit 1 bis 6 Kohlenstoffatomen, einem oder zwei Acylresten mit 2 bis 10 Kohlenstoffatomen, der Carbobenzyloxy- oder tert-Butyloxycarbonylgruppe; |
| $A_1$ | eine Bindung oder ein Peptid mit 1 bis 5 Aminosäureresten ist; |
| $A_2$ | Phe, substituiertes Phenylalanin (SubPhe), β-(2-und 3-Thienyl)alanin, β-(2- und 3-Furanyl)alanin, β-(2-, 3- und 4-Pyridyl)alanin, β-(Benzothien-2- und 3-yl)alanin, β-(1- und 2-Naphthyl)alanin, Tyr oder Trp ist; |
| $A_3$ | Glu oder Asp ist; |
| $A_4$ | irgendeine Aminosäure ist; |
| $A_5$ | Ile, Val, Leu, Nle oder Thr ist; |
| $A_7$ | irgendeine Aminosäure ist; |
| $A_5$ | Glu oder Asp ist; |

| | |
|---|---|
| $A_{10}$ | eine lipophile Aminosäure, ausgewählt aus Tyr, Tyr($SO_3H$), Trp, Phe, Leu, Nle, Ile, Val, His und Pro, oder ein Dipeptid, das mindestens eine dieser lipophilen Aminosäuren enthält, ist; |
| $A_{11}$ | eine Bindung oder ein Peptidfragment mit 1 bis 5 Resten irgendwelcher Aminosäuren ist; |
| Y | einen Rest an der endständigen Carboxygruppe darstellt, ausgewählt aus -OH, einem ($C_1$-$C_6$)Alkoxy-, Amino-, Mono- oder Di-($C_1$-$C_4$)alkylsubstituierten Amino- oder Benzylaminorest; |
| R, R', $R_1$ und $R_1$' | jeweils ausgewählt sind aus einem Wasserstoffatom oder einem ($C_1$-$C_4$)-Alkylrest; |
| B | ausgewählt ist aus den Gruppen -S-, -S-S- oder -S-$Alk_3$-S-; |
| $Alk_1$, $Alk_2$ und $Alk_3$ | jeweils ausgewählt sind aus einer ($C_1$-$C_8$)Methylen- oder Ethylengruppe; |

und in der "D" und "L" anzeigen, daß die Stereochemie des betreffenden Kohlenstoffatoms diejenige ist, die der von D-Cystein bzw. L-Cystein entspricht; und die pharmazeutisch verträglichen Salze davon.

**2.** Peptidderivat gemäß Anspruch 1, in dem $A_2$ Phe, $\beta$-(2-oder 3-Thienyl)alanin oder Tyr ist.

**3.** Peptidderivat gemäß den Ansprüchen 1 oder 2, in dem $A_3$ Glu ist.

**4.** Peptidderivat gemäß einem der Ansprüche 1 bis 3, in dem $A_4$ Glu ist.

**5.** Peptidderivat gemäß einem der Ansprüche 1 bis 4, in dem $A_5$ Ile ist.

**6.** Peptidderivat gemäß einem der Ansprüche 1 bis 5, in dem $A_7$ Glu oder Ala ist.

**7.** Peptidderivat gemäß einem der Ansprüche 1 bis 6, in dem $A_5$ Glu oder Asp ist.

**8.** Peptidderivat gemäß einem der Ansprüche 1 bis 7, in dem $A_{10}$ Leu ist.

**9.** Peptidderivat gemäß einem der Ansprüche 1 bis 8, in dem $A_{11}$ Pro, Gln, Asp oder Asp-Gln ist.

**10.** Peptidderivat gemäß einem der Ansprüche 1 bis 9, in dem X ein Wasserstoffatom, eine Acetyl- oder Bernsteinsäuregruppe ist.

**11.** Peptidderivat gemäß einem der Ansprüche 1 bis 10, in dem Y eine der Gruppen OH oder $NH_2$ ist.

**12.** Peptidderivat gemäß einem der Ansprüche 1 bis 11, in dem die Reste R, R', $R_1$, $R_1$' jeweils ein Wasserstoffatom sind.

**13.** Peptidderivat gemäß einem der Ansprüche 1 bis 12, in dem B eine Gruppe -S-S- ist.

**14.** Peptidderivat gemäß einem der Ansprüche 1 bis 13, in dem $Alk_1$ und $Alk_2$ jeweils eine Methylengruppe der Formel -($CH_2$)- sind.

**15.** Dimer eines Peptidderivats gemäß einem der Ansprüche 1 bis 14 oder ein Gemisch aus dem Dimeren und besagtem Peptid.

**16.** Verfahren zur Herstellung eines Peptidderivats gemäß einem der Ansprüche 1 bis 14 oder eines Dimeren davon oder eines Gemisches des Dimeren und des besagten Polypeptids, das die Erzeugung des freien Sulfhydryl enthaltenden linearen Peptids durch eine Festphasen-Sequenz-oder Blocksynthese, Genclonierung, oder eine Kombination davon und anschließend die oxidative Kupplung des linearen Peptids umfaßt.

**17.** Festphasen-Sequenz- oder Blocksynthese-Verfahren zur Herstellung eines Peptidderivats gemäß einem der Ansprüche 1 bis 14 oder eines Dimeren davon oder eines Gemisches des Dimeren und des besagten Polypeptids, das das Anbinden einer in geeigneter Weise geschützten Aminosäure der Formel $A_1$, die die in Anspruch 1 angegebene Bedeutung hat, an einen aktivierten Harzträger, nachfolgend das Anbinden der anderen $\alpha$-Amino-geschützten Aminosäuren der Formeln $A_2$ bis $A_{11}$, die

die in den vorangegangenen Ansprüchen angegebene Bedeutung haben, an die endständige Aminogruppe der wachsenden Peptidkette, die unterdessen durch Entfernen ihrer Aminoschutzgruppe freigesetzt wurde, und schließlich die oxidative Kupplung des linearen Peptids umfaßt.

**18.** Peptidderivat gemäß einem der Ansprüche 1 bis 14, ein Dimer davon oder ein Gemisch dieser Verbindungen zur Verwendung als Arzneistoff.

**19.** Peptidderivat gemäß einem der Ansprüche 1 bis 14, ein Dimer davon oder ein Gemisch dieser Verbindungen zur Verwendung als Antikoagulierungsmittel.

**Revendications**
**Revendications pour les Etats contractants suivants : AT, BE, CH, DE, FR, GB, IT, LI, LU, NL, SE**

**1.** Dérivé de peptide de formule générale 1:

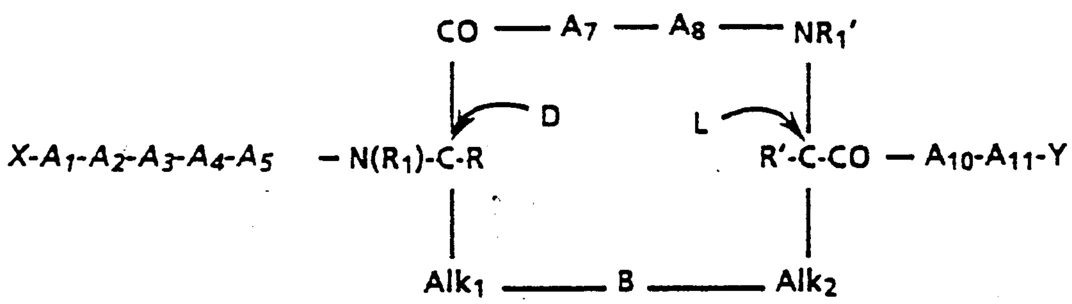

1

dans laquelle

X est un résidu de terminaison amino choisi parmi l'hydrogène, un ou deux groupes alkyle de 1 à 6 atomes de carbone, un ou deux groupes acyle de 2 à 10 atomes de carbone, carbobenzyloxy, ou t-butyloxycarbonyle;

$A_1$ est une liaison ou un peptide contenant de 1 à 5 résidus d'aminoacides quelconques;

$A_2$ est Phe, phénylalanine substituée (SubPhe), $\beta$-(2- et 3-thiényl)alanine, $\beta$-(2- et 3-furanyl)alanine, $\beta$-(2-, 3-, et 4-pyridyl)alanine, $\beta$-(benzothién-2- et 3-yl)alanine, $\beta$-(1-et 2-naphtyl)alanine, Tyr ou Trp;

$A_3$ est Glu ou Asp;

$A_4$ est un aminoacide quelconque;

$A_5$ est Ile, Val, Leu, Nle, ou Thr;

$A_7$ est un aminoacide quelconque;

$A_5$ est Glu ou Asp;

$A_{10}$ est un aminoacide lipophile choisi parmi Tyr, Tyr($SO_3H$), Trp, Phe, Leu, Nle, Ile, Val, His, et Pro, ou est un dipeptide contenant au moins l'un de ces aminoacides lipophiles;

$A_{11}$ est une liaison ou est un fragment de peptide contenant de un à cinq résidus d'aminoacides quelconques;

Y est un résidu de terminaison carboxy choisi parmi OH, alcoxy en $C_1$-$C_6$, amino, amino mono- ou disubstitué par alkyle en $C_1$-$C_4$, ou benzylamino;

R, R', $R_1$ et $R_1$' sont choisis chacun parmi un hydrogène et un groupe alkyle en $C_1$-$C_4$;

B est choisi parmi -S-, -S-S-, ou -S-$Alk_3$-S-;

$Alk_1$, $Alk_2$ et $Alk_3$ sont choisis chacun parmi un groupe éthylène ou méthylène en $C_1$-$C_8$;

et dans laquelle "D" et "L" indiquent que la stéréochimie du carbone indiqué est celle correspondant respectivement à la D-cystéine et à la L-cystéine, et leurs sels pharmaceutiquement acceptables.

**2.** Dérivé de peptide selon la revendication 1, dans lequel $A_2$ est Phe, $\beta$-(2- ou 3-thiényl)alanine, ou Tyr.

**3.** Dérivé de peptide selon la revendication 1 ou 2, dans lequel $A_3$ est Glu.

**4.** Dérivé de peptide selon l'une quelconque des revendications 1 à 3, dans lequel $A_4$ est Glu.

**5.** Dérivé de peptide selon l'une quelconque des revendications 1 à 4, dans lequel $A_5$ est Ile.

6. Dérivé de peptide selon l'une quelconque des revendications 1 à 5, dans lequel $A_7$ est Glu ou Ala.

7. Dérivé de peptide selon l'une quelconque des revendications 1 à 6, dans lequel $A_8$ est Glu ou Asp.

8. Dérivé de peptide selon l'une quelconque des revendications 1 à 7, dans lequel $A_{10}$ est Leu.

9. Dérivé de peptide selon l'une quelconque des revendications 1 à 8, dans lequel $A_{11}$ est Pro, Gln, Asp ou Asp-Gln.

10. Dérivé de peptide selon l'une quelconque des revendications 1 à 9, dans lequel X est H, acétyle ou succinyle.

11. Dérivé de peptide selon l'une quelconque des revendications 1 à 10, dans lequel Y est OH ou $NH_2$.

12. Dérivé de peptide selon l'une quelconque des revendications 1 à 11, dans lequel R, R', $R_1$, $R_1$' sont chacun un hydrogène.

13. Dérivé de peptide selon l'une quelconque des revendications 1 à 12, dans lequel B est le groupe -S-S-.

14. Dérivé de peptide selon l'une quelconque des revendications 1 à 13, dans lequel $Alk_1$ et $Alk_2$ sont chacun un groupe méthylène de formule $-(CH_2)-$.

15. Dimère d'un dérivé de peptide selon l'une quelconque des revendications 1 à 14 ou mélange du dimère et dudit peptide.

16. Procédé de préparation d'un dérivé de peptide selon l'une quelconque des revendications 1 à 14, ou d'un de ses dimères ou d'un mélange du dimère et dudit polypeptide, dans lequel on prépare le peptide linéaire contenant des groupes sulfhydryle libres par une synthèse séquentielle en phase solide ou en bloc, un clonage de gènes, ou une combinaison de ces méthodes, et on soumet ensuite le peptide lineaire à un couplage oxydant.

17. Procédé de synthèse séquentielle en phase solide ou en bloc pour la préparation d'un dérivé de peptide selon l'une quelconque des revendications 1 à 14, d'un de ses dimères ou d'un mélange du dimére ou d'un mélange du dimère et dudit polypeptide, dans lequel on lie un aminoacide de formule $A_1$ ayant la signification donnée dans la revendication 1, convenablement protégé, à un support résinique activé, puis on lie les autres aminoacides de formule $A_2$ à $A_{11}$ ayant la signification donnée dans les revendications précédentes, dont le groupe amino en alpha est protégé, au groupe amino terminal de la chaîne peptidique croissante qu'on a exposé entre temps en éliminant son groupe protecteur d'amino, et on soumet finalement le peptide linéaire à un couplage oxydant.

18. Dérivé de peptide selon l'une quelconque des revendications 1 à 14, dimère de ce dérivé, ou mélange de ces composés, pour l'utilisation comme composé pharmaceutiquement actif.

19. Dérivé de peptide selon l'une quelconque des revendications 1 à 14, dimère de ce dérivé, ou mélange de ces composés, pour l'utilisation comme anticoagulant.

20. Composition pharmaceutique contenant une quantité efficace comme anticoagulant d'un dérivé de peptide selon l'une quelconque des revendications 1 à 14, d'un de ses dimères, ou d'un mélange de ces composés, et éventuellement un support et/ou un diluant pharmaceutiquement acceptables.

**Revendications pour l'Etat contractant suivant : ES**

1. Procédé de préparation d'un dérivé de peptide de formule

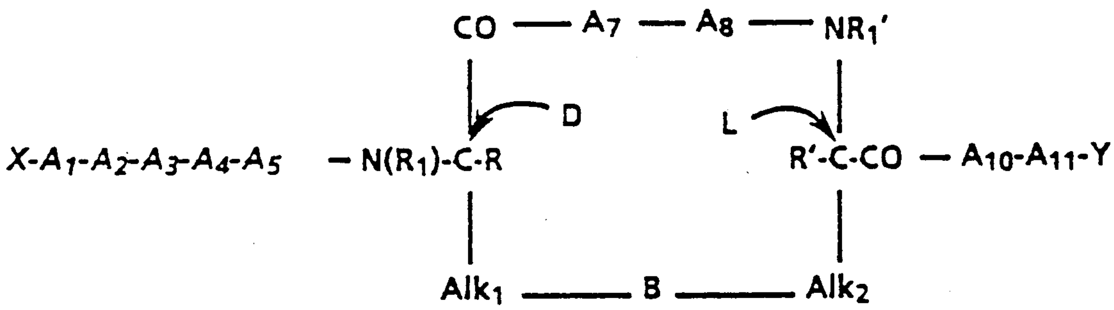

dans laquelle

X est un résidu de terminaison amino choisi parmi l'hydrogène, un ou deux groupes alkyle de 1 à 6 atomes de carbone, un ou deux groupes acyle de 2 à 10 atomes de carbone, carbobenzyloxy, ou t-butyloxycarbonyle;

$A_1$ est une liaison ou un peptide contenant de 1 à 5 résidus d'aminoacides quelconques;

$A_2$ est Phe, phénylalanine substituée (SubPhe), $\beta$-(2- et 3-thiényl)alanine, $\beta$-(2- et 3-furanyl)alanine, $\beta$-(2-, 3-, et 4-pyridyl)alanine, $\beta$-(benzothién-2- et 3-yl)alanine, $\beta$-(1-et 2-naphtyl)alanine, Tyr ou Trp;

$A_3$ est Glu ou Asp;

$A_4$ est un aminoacide quelconque;

$A_5$ est Ile, Val, Leu, Nle, ou Thr;

$A_7$ est un aminoacide quelconque;

$A_5$ est Glu ou Asp;

$A_{10}$ est un aminoacide lipophile choisi parmi Tyr, Tyr($SO_3H$), Trp, Phe, Leu, Nle, Ile, Val, His, et Pro, ou est un dipeptide contenant au moins l'un de ces aminoacides lipophiles;

$A_{11}$ est une liaison ou est un fragment de peptide contenant de un à cinq résidus d'aminoacides quelconques;

Y est un résidu de terminaison carboxy choisi parmi OH, alcoxy en $C_1$-$C_6$, amino, amino mono- ou disubstitué par alkyle en $C_1$-$C_4$, ou benzylamino;

R, R', $R_1$ et $R_1$' sont choisis chacun parmi un hydrogène et un groupe alkyle en $C_1$-$C_4$;

B est choisi parmi -S-, -S-S-, ou -S-$Alk_3$-S-;

$Alk_1$, $Alk_2$ et $Alk_3$ sont choisis chacun parmi un groupe éthylène ou méthylène en $C_1$-$C_8$;

et dans laquelle "D" et "L" indiquent que la stéréochimie du carbone indiqué est celle correspondant respectivement à la D-cystéine et à la L-cystéine, dans lequel on prépare le peptide linéaire contenant des groupes sulfhydryle libres par une synthèse séquentielle en phase solide ou en bloc, un clonage de gènes, ou une combinaison de ces méthodes, et on soumet ensuite le peptide linéaire à un couplage oxydant.

2. Procédé de synthèse séquentielle en phase solide ou en bloc pour la préparation d'une dérivé de peptide de formule

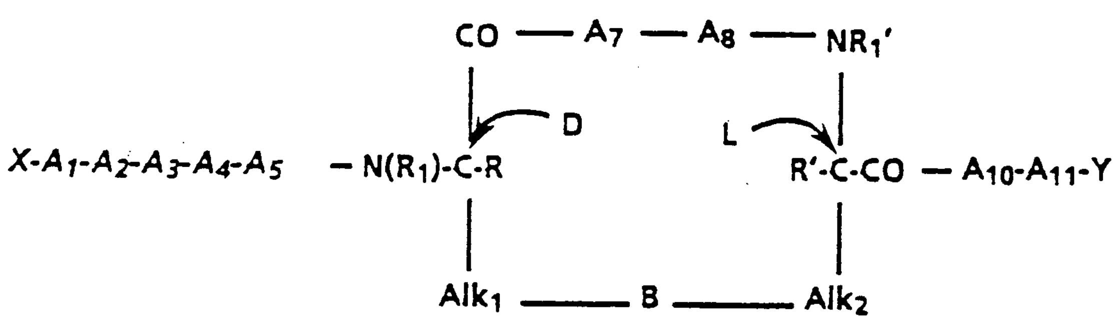

dans laquelle

X est un résidu de terminaison amino choisi parmi l'hydrogène, un ou deux groupes alkyle de 1 à 6 atomes de carbone, un ou deux groupes acyle de 2 à 10 atomes de carbone, carbobenzyloxy, ou t-butyloxycarbonyle;

$A_1$ est une liaison ou un peptide contenant de 1 à 5 résidus d'aminoacides quelconques;

$A_2$ est Phe, SubPhe, $\beta$-(2- et 3-thiényl)alanine, $\beta$-(2- et 3-furanyl)alanine, $\beta$-(2-, 3-, et 4-pyridyl)-alanine, $\beta$-(benzothién-2- et 3-yl)alanine, $\beta$-(1- et 2-naphtyl)alanine, Tyr ou Trp;

$A_3$ est Glu ou Asp;

$A_4$ est un aminocide quelconque;

$A_5$ est Ile, Val, Leu, Nle, ou Thr;

$A_7$ est un aminoacide quelconque;

$A_5$ est Glu ou Asp;

$A_{10}$ est un aminoacide lipophile choisi parmi Tyr, Tyr($SO_3H$), Trp, Phe, Leu, Nle, Ile, Val, His, et Pro, ou est un, dipeptide contenant au moins l'un de ces aminoacides lipophiles;

$A_{11}$ est une liaison ou est un fragment de peptide contenant de un à cinq résidus d'aminoacides quelconques;

Y est un résidu de terminaison carboxy choisi parmi OH, alcoxy en $C_1$-$C_6$, amino, amino mono- ou disubstitué par alkyle en $C_1$-$C_4$, ou benzylamino;

R, R', $R_1$ et $R_1$' sont choisis chacun parmi un hydrogène et un groupe alkyle en $C_1$-$C_4$;

B est choisi parmi -S-, -S-S-, ou -S-$Alk_3$-S-;

$Alk_1$, $Alk_2$ et $Alk_3$ sont choisis chacun parmi un groupe éthylène ou méthylène en $C_1$-$C_8$;

et dans laquelle "D" et "L" indiquent que la stéréochimie du carbone indiqué est celle correspondant respectivement à la D-cystéine et à la L-cystéine, dans lequel on lie un aminoacide de formule $A_1$ convenablement protégé à un support résinique activé, puis on lie les autres aminoacides de formule $A_2$ à $A_{11}$, dont le groupe amino en alpha est protégé, au groupe amino terminal de la chaîne peptidique croissante qu'on a exposé entre temps en éliminant son groupe protecteur d'amino, et on soumet finalement le peptide linéaire à un couplage oxydant.

**3.** Procédé selon la revendication 1 ou 2, qui comprend en plus la formation d'un dimère du dérivé de peptide obtenu.

**4.** Procédé selon l'une quelconque des revendications 1 à 3, dans lequel $A_2$ est Phe, $\beta$-(2- ou 3-thiényl)-alanine, ou Tyr.

**5.** Procédé selon l'une quelconque des revendications 1 à 4, dans lequel $A_3$ est Glu.

**6.** Procédé selon l'une quelconque des revendications 1 à 5, dans lequel $A_4$ est Glu.

**7.** Procédé selon l'une quelconque des revendications 1 à 6, dans lequel $A_5$ est Ile.

**8.** Procédé selon l'une quelconque des revendications 1 à 7, dans lequel $A_7$ est Glu ou Ala.

**9.** Procédé selon l'une quelconque des revendications 1 à 8, dans lequel $A_5$ est Glu ou Asp.

**10.** Procédé selon l'une quelconque des revendications 1 à 9, dans lequel $A_{10}$ est Leu.

**11.** Procédé selon l'une quelconque des revendications 1 à 10, dans lequel $A_{11}$ est Pro, Gln, Asp ou Asp-Gln.

**12.** Procédé selon l'une quelconque des revendications 1 à 11, dans lequel X est H, acétyle ou succinyle.

**13.** Procédé selon l'une quelconque des revendications 1 à 12, dans lequel Y est OH ou $NH_2$.

**14.** Procédé selon l'une quelconque des revendications 1 à 13, dans lequel R, R', $R_1$, $R_1$' sont chacun un hydrogène.

**15.** Procédé selon l'une quelconque des revendications 1 à 14, dans lequel B est le groupe -S-S-.

**16.** Procédé selon l'une quelconque des revendications 1 à 15, dans lequel $Alk_1$ et $Alk_2$ sont chacun un groupe méthylène de formule $-(CH_2)-$.

**17.** Utilisation d'un composé pouvant être obtenu suivant le procédé de l'une quelconque des revendications 1 à 16 pour la préparation d'une composition pharmaceutique.

**18.** Utilisation d'un composé pouvant être obtenu suivant le procédé de l'une quelconque des revendications 1 à 16 pour la préparation d'un anticoagulant.

**Revendications pour l'Etat contractant suivant : GR**

**1.** Dérivé de peptide de formule générale 1:

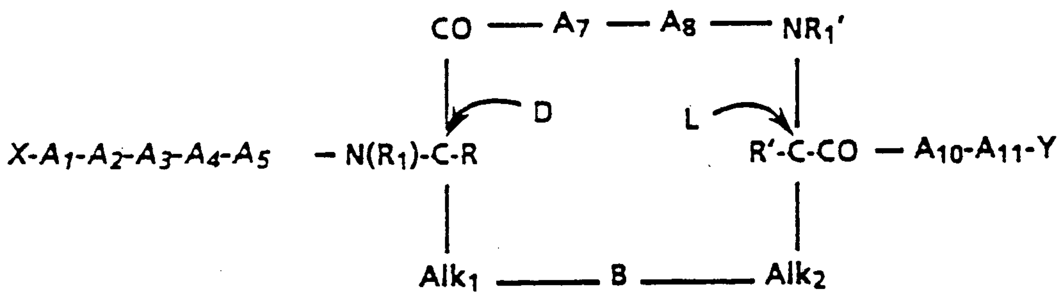

1

dans laquelle

X est un résidu de terminaison amino choisi parmi l'hydrogène, un ou deux groupes alkyle de 1 à 6 atomes de carbone, un ou deux groupes acyle de 2 à 10 atomes de carbone, carbobenzyloxy, ou t-butyloxycarbonyle;

$A_1$ est une liaison ou un peptide contenant de 1 à 5 résidus d'aminoacides quelconques;

$A_2$ est Phe, phénylalanine substituée (SubPhe), $\beta$-(2- et 3-thiényl)alanine, $\beta$-(2- et 3-furanyl)alanine, $\beta$-(2-, 3-, et 4-pyridyl)alanine, $\beta$-(benzothién-2- et 3-yl)alanine, $\beta$-(1-et 2-naphtyl)alanine, Tyr ou Trp;

$A_3$ est Glu ou Asp;

$A_4$ est un aminoacide quelconque;

$A_5$ est Ile, Val, Leu, Nle, ou Thr;

$A_7$ est un aminoacide quelconque;

$A_5$ est Glu ou Asp;

$A_{10}$ est un aminoacide lipophile choisi parmi Tyr, Tyr($SO_3H$), Trp, Phe, Leu, Nle, Ile, Val, His, et Pro, ou est un dipeptide contenant au moins l'un de ces aminoacides lipophiles;

$A_{11}$ est une liaison ou est un fragment de peptide contenant de un à cinq résidus d'aminoacides quelconques;

Y est un résidu de terminaison carboxy choisi parmi OH, alcoxy en $C_1$-$C_6$, amino, amino mono- ou disubstitué par alkyle en $C_1$-$C_4$, ou benzylamino;

R, R', $R_1$ et $R_1$' sont choisis chacun parmi un hydrogène et un groupe alkyle en $C_1$-$C_4$;

B est choisi parmi -S-, -S-S-, ou -S-$Alk_3$-S-;

$Alk_1$, $Alk_2$ et $Alk_3$ sont choisis chacun parmi un groupe éthylène ou méthylène en $C_1$-$C_8$;

et dans laquelle "D" et "L" indiquent que la stéréochimie du carbone indiqué est celle correspondant respectivement à la D-cystéine et à la L-cystéine, et ses sels pharmaceutiquement acceptables.

**2.** Dérivé de peptide selon la revendication 1, dans lequel $A_2$ est Phe, $\beta$-(2- ou 3-thiényl)alanine, ou Tyr.

**3.** Dérivé de peptide selon la revendication 1 ou 2, dans lequel $A_3$ est Glu.

**4.** Dérivé de peptide selon l'une quelconque des revendications 1 à 3, dans lequel $A_4$ est Glu.

**5.** Dérivé de peptide selon l'une quelconque des revendications 1 à 4, dans lequel $A_5$ est Ile.

**6.** Dérivé de peptide selon l'une quelconque des revendications 1 à 5, dans lequel $A_7$ est Glu ou Ala.

7. Dérivé de peptide selon l'une quelconque des revendications 1 à 6, dans lequel $A_8$ est Glu ou Asp.

8. Dérivé de peptide selon l'une quelconque des revendications 1 à 7, dans lequel $A_{10}$ est Leu.

9. Dérivé de peptide selon l'une quelconque des revendications 1 à 8, dans lequel $A_{11}$ est Pro, Gln, Asp ou Asp-Gln.

10. Dérivé de peptide selon l'une quelconque des revendications 1 à 9, dans lequel X est H, acétyle ou succinyle.

11. Dérivé de peptide selon l'une quelconque des revendications 1 à 10, dans lequel Y est OH ou $NH_2$.

12. Dérivé de peptide selon l'une quelconque des revendications 1 à 11, dans lequel R, R', $R_1$, $R_1'$ sont chacun un hydrogène.

13. Dérivé de peptide selon l'une quelconque des revendications 1 à 12, dans lequel B est le groupe -S-S-.

14. Dérivé de peptide selon l'une quelconque des revendications 1 à 13, dans lequel $Alk_1$ et $Alk_2$ sont chacun un groupe méthylène de formule $-(CH_2)-$.

15. Dimère d'un dérivé de peptide selon l'une quelconque des revendications 1 à 14 ou mélange du dimère et dudit peptide.

16. Procédé de préparation d'un dérivé de peptide selon l'une quelconque des revendications 1 à 14, ou d'un de ses dimères ou d'un mélange du dimère et dudit polypeptide, dans lequel on prépare le peptide linéaire contenant des groupes sulfhydryle libres par une synthèse séquentielle en phase solide ou en bloc, un clonage de gènes, ou une combinaison de ces méthodes, et on soumet ensuite le peptide linéaire à un couplage oxydant.

17. Procédé de synthèse séquentielle en phase solide ou en bloc pour la préparation d'un dérivé de peptide selon l'une quelconque des revendications 1 à 14, d'un de ses dimères ou d'un mélange du dimère et dudit polypeptide, dans lequel on lie un aminoacide de formule $A_1$ ayant la signification donnée dans la revendication 1, convenablement protégé, à un support résinique activé, puis on lie les autres aminoacides de formule $A_2$ à $A_{11}$ ayant la signification donnée dans les revendications précédentes, dont le groupe amino en alpha est protégé, au groupe amino terminal de la chaîne peptidique croissante qu'on a exposé entre temps en éliminant son groupe protecteur d'amino, et on soumet finalement le peptide linéaire à un couplage oxydant.

18. Dérivé de peptide selon l'une quelconque des revendications 1 à 14, dimère de ce dérivé, ou mélange de ces composés, pour l'utilisation comme composé pharmaceutiquement actif.

19. Dérivé de peptide selon l'une quelconque des revendications 1 à 14, dimère de ce dérivé, ou mélange de ces composés, pour l'utilisation comme anticoagulant.